# EUROPEAN PATENT SPECIFICATION

(11) **EP 4 210 659 B1**
(45) Date of publication and mention of the grant of the patent: **14.05.2025**
(21) Application number: 21777958.6
(22) Date of filing: 09.09.2021
(51) Int. Cl.: A61K 8/25, A61Q 17/04, A61K 8/9706

(54) **BENTHIC PENNATE DIATOM FRUSTULES AS AN SPF BOOSTER**
BENTHISCHE GEFIEDERTE KIESELALGENFRUSTELN ALS SPF-BOOSTER
FRUSTULES DE DIATOMÉES DE PENNATE BENTHIQUE EN TANT QU'AMPLIFICATEUR DE FPS

(30) Priority: 14.09.2020 EP 20195947
(43) Date of publication of application: 19.07.2023
(73) Proprietor: Swedish Algae Factory AB, 416 64 Göteborg (SE)
(72) Inventor: ALLERT, Sofie, 42363 Torslanda (SE); ANDRÉN, Lizette, 41304 Göteborg (SE); ROVA, Emma, 41669 Göteborg (SE)
(74) Representative: Henricson Briggs, David James
(86) International application number: PCT/EP2021/074754
(87) International publication number: WO 2022/053539

(56) References cited:
- WO-A1-2017/211892
- FR-A1- 3 054 128
- US-A1- 2020 232 003

## Description

### Field of the Invention

The present disclosure relates to compositions comprising benthic pennate diatom frustules as an SPF Booster. In particular it relates to compositions comprising a sunscreen active agent and benthic pennate diatom frustules.

### Background of the invention

Sunscreens are used as cosmetic products to protect the skin from harm caused by UV radiation. A common measure for determining the protective value of a sunscreen is the Sun Protection Factor (SPF) of the sunscreen. Sun Protection Factor (SPF) is defined as the ratio of the amount of energy required to produce a minimal erythema on sunscreen protected skin to the amount of energy required to produce the same level of erythema on unprotected skin.

The UV radiation causing damage to the skin may be both UVA and UVB rays.

Cosmetic products having an increased or boosted SPF are important to provide better protection to the skin from the harmful UV radiation. Physical UV blockers and/or chemical UV absorbers are used as active sunscreen agents to provide protection from UV radiation. However, some active sunscreen agents have been reported to have adverse toxicological effects. Furthermore, an increased amount of an active sunscreen agent may lead to less desirable cosmetic properties, for example, a paste-like consistency, or lead to skin-whitening.

SPF boosters have been developed to increase the UV protection factor of sunscreens without the addition of increased amount of active sunscreen agent. Or similarly, to allow a reduction in the amount of active sunscreen agent whilst maintaining a similar level of UV protection, i.e., SPF. An SPF Booster need not have a UV protecting effect itself but rather increases the SPF of a cosmetic composition comprising an active sunscreen agent.

FR 3 054 128 A1 (CODIF International) describes a cosmetic composition comprising biomineral compounds such as coccoliths and/or non-specific diatom frustules and contends that such compositions reduce UV induced inflammatory biomarkers in human skin. FR 3 054 128 A1 does not disclose an SPF booster or any SPF, UV blocking/screening effect of the biomineral compounds tested, but rather the post UV exposure anti-inflammatory effects of a cream.

EP 3 658 107 A1 (Dow Global Technologies LLC) describes an SPF booster for use in alcohol-based sunscreen formulations. The SPF booster is a multi-stage polymeric particle.

Improved, naturally sourced SPF boosters having a proven SPF booster effect would be ideal to meet consumer expectations regarding performance and the ingredients of cosmetic products.

### Summary of the invention

Accordingly, the present invention preferably seeks to mitigate, alleviate or eliminate one or more of the above-identified deficiencies in the art and disadvantages singly or in any combination and solves at least the above mentioned problems by providing a cosmetic composition comprising: at least one sunscreen active agent; and, benthic pennate diatom frustules.

A method of boosting the SPF of a sunscreen is provided.

A method of preparing a sunscreen comprising benthic pennate diatom frustules is also provided.

Further advantageous embodiments are disclosed in the appended and dependent patent claims.

### Brief description of the figures

The figures show results from the experiments described in the experimental section.
Fig. 1 is the MPF values obtained for a Reference SPF composition. The figure shows 9 scans and an average MPF for a typical plate of the 5 scanned plates.
Fig. 2 is the MPF values obtained for a control composition comprising no benthic pennate diatoms. The figure shows 9 scans and an average MPF for a typical plate of the 5 scanned plates.
Fig. 3 is the MPF values obtained for a composition comprising 0.1% benthic pennate diatoms. The figure shows 9 scans and an average MPF for a typical plate of the 5 scanned plates.
Fig. 4 is the MPF values obtained for a composition comprising 0.3% benthic pennate diatoms. The figure shows 9 scans and an average MPF for a typical plate of the 5 scanned plates.

### Detailed description

The present disclosure relates to a cosmetic composition comprising at least one sunscreen active agent and benthic pennate diatom frustules. The benthic pennate diatom frustules boost the protective value of the sunscreen (SPF) compared to compositions comprising no benthic pennate diatom frustules. The benthic pennate diatom frustules are thereby an SPF booster.

Diatoms are a type of algae which are present in both fresh water and marine environments. The diatoms comprise a frustule which has a silicon dioxide content. The frustules are a source of nanoporous silicon dioxide. Benthic diatoms are diatoms which grow attached to surfaces at the water-sediment interface, this is as opposed to pelagic diatoms which grow in open water. Pennate diatoms are diatoms which are bilaterally symmetrical as opposed to radially symmetrical diatoms, which are defined as centric diatoms.

The light manipulating properties of benthic pennate diatom frustules have been previously noted in solar cell applications where they enhance the efficiency of solar cells. See for example, WO 2017/211892 A1 (Swedish Algae Factory AB). However, there has been to-date no suggestion that benthic pennate diatoms may be used in cosmetic compositions as an SPF booster.

Cosmetic compositions comprising fossilised frustules, that is diatomaceous earth, are known in the field. The diatomaceous earth is used in such processes as bulking, opacifying or anti-caking agent. In use as, for example, and opacifying agent the diatomaceous earth may have a UV absorbing effect. As opposed to fossilised diatom frustules, i.e., diatomaceous earth, benthic pennate diatom frustules have an SPF boosting effect which is significantly greater than that which could be expected for a similar amount of diatomaceous earth. This is evidenced by the experimental section below, where a cosmetic composition comprising only 0.1% benthic pennate diatoms had an almost 22% greater SPF than the control composition comprising no benthic pennate diatoms.

Without wishing to be bound by theory the inventors contend that the specific structure and form of the benthic pennate diatom frustules has both a UV absorbing, UV refracting, and UV conversion effect. The UV conversion effect may be the conversion of UV light from UV wavelengths to light in the visible spectrum (e.g., blue) via silica photoluminescence. Such SPF boosting activity has not been shown in fossilised diatom frustules. Centric pelagic diatoms frustules of *C. wailesii* have been shown to emit blue light when excited with light at a wavelength of 325 nm (De Tommasi, E. et al. UV-shielding and wavelength conversion by centric diatom nanopatterned frustules. Sci Rep 8, 16285 (2018)). As benthic pennate diatoms are less exposed to UV radiation in the natural environment it is generally considered that they are less likely to exhibit UV protective capabilities and rather evolved to migrate downwards, away from UV radiation. (De Tommasi, E. et al. Sci Rep 8, 16285 (2018)). The present results indicate that benthic pennate diatom frustules have an SPF boosting activity which was not expected from benthic pennate diatom frustules. It is thus contended that the UV conversion process may be occurring with the benthic pennate diatoms in the present composition.

The composition may comprise at least 0.05% by weight benthic pennate diatom frustules. The composition may comprise from 0.1% to about 3% by weight benthic pennate diatom frustules. The composition may comprise from about 0.05% to about 1% by weight, such as from about 0.1% to about 1% by weight, benthic pennate diatom frustules. The composition may comprise from about 0.1% to about 0.5% by weight benthic pennate diatom frustules, such as from about 0.1% to about 0.3%. The benthic pennate diatom frustules are extracted from diatoms and are not fossil diatom frustules.

The composition may comprise benthic pennate diatom frustules at an amount which forms a monolayer when applied to a surface. The term monolayer of frustules means that there is limited, such as no, overlap between diatom frustules. The benthic pennate diatom frustules may self-arrange into a monolayer when provided at a sufficiently low concentration.

The frustules may be extracted from cultured and harvested benthic pennate diatoms in accordance with the process described in WO 2017/211892 A1 (Swedish Algae Factory AB). The frustules may be extracted via a chemical extraction process whereby the organic biomass of the diatom is removed via a chemical agent leaving the frustules.

The present composition comprises at least one sunscreen active agent. The at least one sunscreen active agent may be an inorganic sunscreen active agent or an organic sunscreen active agent, or a combination thereof. The composition may comprise a plurality of different inorganic or organic sunscreen active agents. The compositions PE1 to PE3 of the experimental section comprise a plurality of physical blocking inorganic sunscreen active agents. The combination of different active agents may provide improved sun protective power over a range of UV wavelengths.

Inorganic sunscreen active agents may be for example, zinc oxide, titanium dioxide, iron oxide, zirconium oxide, cerium oxide. Inorganic sunscreen active agents may be nanopigments formed from coated metal oxides as those disclosed herein. Preferably the inorganic sunscreen active agent is zinc oxide, titanium dioxide or a mixture thereof. The inorganic sunscreen active may be a transparent inorganic metal oxide, such as transparent zinc oxide or transparent titanium dioxide. As is known in the art, transparent inorganic metal oxides are metal oxides which have been prepared in such a manner that they appear transparent to visible light and therefore do not appear white when applied to the skin. For the sake of clarification, the inorganic sunscreen agent may be a transparent metal oxide or a non-transparent metal oxide.

The sunscreen active agent may be an organic sunscreen active agent, such as anthranilates, cinnamic derivatives, dibenzoylmethane derivatives, salicylic derivatives, camphor derivatives, triazine derivatives, benzophenone derivatives, β,β-diphenylacrylate derivatives, benzotriazole derivatives, benzalmalonate derivatives, benzimidazole derivatives, imidazolines, bisbenzoazolyl derivatives, p-aminobenzoic acid (PABA) derivatives, methylenebis(hydroxyphenylbenzotriazole) derivatives, screening polymers and screening silicones, dimers derived from α-alkylstyrene, 4,4-diarylbutadienes; and their mixtures.

Benthic pennate diatom frustules have the advantage over other known SPF boosters such as polymer-based SPF boosters in that they are natural, i.e., they are naturally occurring. Cosmetic products with natural ingredients are often preferred by users as they are considered less harmful to the body, and/or to the environment. The benthic pennate diatoms may be extracted from cultured diatoms grown according to the process described in WO 2017/211892 A1 (Swedish Algae Factory AB). The benthic pennate diatoms may be cultured in, water comprising at least a portion of waste-water from for example, fish farms or food processing plants. Such waste-water has an increased nitrogen content which may be ideal for culturing the diatoms. Such a manufacturing process provides an SPF booster which is both an effective SPF booster and a means to recycle waste-water. To this end, the benthic pennate diatom frustules are especially suitable as SPF boosters in sunscreen compositions comprising inorganic, UV blocking sunscreen active agents, as such sunscreen compositions are often marketed as natural sunscreens or chemical free sunscreens. As would be understood, "chemical free" in this context refers to the lack of chemical absorbing agents such as organic sunscreen active agents, for example p-aminobenzoic acid (PABA) derivatives.

The sunscreen active agents absorb radiation in the UVA and/or UVB spectrum. The UVA spectrum is the 280 nm to 320 nm region of the ultraviolet light spectrum. The UVB spectrum is the 320 nm to 400 nm region of the ultraviolet light spectrum.

The at least one sunscreen active agent is generally present in an amount from about 0.1% to about 75% by weight of the composition. The sunscreen active agent may be present in an amount from about 1% to about 40% by weight of the composition, such as about 5% to about 25%. The sunscreen active agent may be present in an amount from about 10% to about 15% by weight of the composition. Due to the presence of the benthic pennate diatom frustules in the composition, the sunscreen active agent may be present in an amount less than generally needed to achieve a specific SPF in a composition.

The compositions may also comprise water and/or oil. The compositions may be an emulsion comprising water. The compositions may be in the form of a water-in-oil emulsion or an oil-in-water emulsion. Water or oil may be present in amounts from about 5% to about 70% by weight of the composition.

The composition may be a powdered sunscreen composition comprising benthic pennate diatom frustules. A powdered or powder sunscreen composition is a composition comprising fine non-agglomerated particles in a carrier medium. Powdered sunscreen compositions are known in the art. Benthic pennate diatom frustules are especially suitable for use in powdered sunscreen compositions as the frustules are a dry powder.

The composition may comprise other ingredients such as emollients, humectants, lubricating agents, conditioning agents, surfactants, plasticizers, preservatives, fillers, active ingredients that are not sunscreen active agents, perfumes, thickeners, antioxidants, vitamins and other ingredients which are known and commonly used in cosmetic compositions. Compositions may comprise metal oxides in addition to the inorganic sunscreen active agents listed above. The compositions may comprise for example, alumina, mica. Compositions comprising benthic pennate diatoms may also comprise silica, separate and in addition to the silicon oxide frustules, for cosmetic application purposes.

### Experimental Section

The boosting efficacy of benthic pennate diatoms to a UV filter composition was determined via *in vitro* studies. The Sun Protection Factor (SPF) of a known composition was compared to the SPF of compositions comprising benthic pennate diatom frustules at various concentrations.

### General description

The test products were spread onto a thin film on a suitable synthetic substrate and the UV absorbance through this film is measured with a spectrophotometer. The sun protection factor was determined by firstly irradiating the product with 4 Minimum Erythemal Doses (MEDs) followed by the scanning of the sample from 290nm to 400nm. During the scanning the obtained data was accumulated and stored at intervals of 1nm to determine the monochromatic protection factor (MPF) for each of the selected wavelengths. Then the MPF is used to calculate the SPF value, using solar irradiance and erythemal constants.

### Sample Preparation

Benthic pennate diatom frustules were added to an existing sunscreen formulation, whereby the water content by weight was adjusted to compensate for the addition of the benthic pennate diatom frustules. The existing sunscreen formulation comprised an inorganic sunscreen active agent.

The following compositions were prepared.

**Table 1: The different compositions PE1-PE3 and the Reference SPF composition**

| Composition | Benthic pennate diatom frustule (% wt.) | Purified Water (% wt.) | Inorganic sunscreen active agent (Zinc oxide, Titanium dioxide, Silica) (% wt.) | Other (% wt.) |
|---|---|---|---|---|
| PE1 (Control, 0%) | 0% | 44.40% | 12% | 43.60% |
| PE2 (0.1%) | 0.1% | 44.30% | 12% | 43.60% |
| PE3 (0.3%) | 0.3% | 44.10% | 12% | 43.60% |
| Reference SPF composition (SPF *in vivo* 16.1 +/- 2.4) | | | | |

**Table 2: Other ingredients of the compositions PE1 to PE3**

| | Amount (% wt.) |
|---|---|
| Preservative (glycerine, aqua, sodium levulinate, sodium anisate) | 4% |
| Glycerine | 2% |
| Magnesium sulphate | 1% |
| Lactic acid | 0.1% |
| Squalane | 8% |
| Seed oils | 12% |
| Silk (Hydrogenated Ethylhexyl Olivate, Hydrogenated Olive Oil Unsaponifiables) | 8% |
| Emulsion (water-in-oil, Polyglyceryl-3 Polyricinoleate; Sorbitan Sesquioleate; Cetyl Ricinoleate; Glyceryl Caprate; Cera Alba; Magnesium Stearate) | 8% |
| Vitamin E | 0.5% |

Moulded PMMA plates, 50mmx50mm, (ref.: HEL-PMMA55 (HelioScience, France) were used as a substrate. The foil covered PMMA plates were uncovered and cleaned with an antistatic cloth. The PMMA plates were weighed. The above compositions (PE1-PE3, SPF reference) comprising benthic pennate diatom frustules at various concentrations were applied to the PMMA plate. Five plates of each of PE1-PE3 were prepared. Four plates of Reference SPF composition were prepared. Approximately 1.3 mg/cm2 of each composition was applied to the surface of the PMMA plate. Total mass of composition on each plate was 32.5 ± 0.3 mg. The compositions were spread over the surface of the plate with a saturated gloved finger. The plates with the compositions applied to the surface were allowed to dry for at least 30 mins in the dark at the same temperature, 30.0±5.0°C and relative humidity of 50.0±10.0%. The same temperature and humidity conditions were used for the UV exposure and absorbance measurements.

### Equipment

The Irradiation source was a Solar Simulator PV Cell testing with model number 16S-300-002 (Solarlight^{®}). The irradiation source was calibrated according to the manufacturer's specifications.

UV spectrophotometer, SPF-290AS (Solarlight^{®}) was used to perform all absorbance measurements. The UV spectrophotometer was calibrated according to the manufacturer's specifications.

UVB radiometer (SUV Detector PMA2101S-UVS, Solarlight^{®}) was calibrated against a spectroradiometric measurement of the solar simulator output.

Dose controller/meter (DCS 2.0 Dose Controller/Meter, Solarlight^{®}) was calibrated according to the manufacturer's specifications.

### UV test of known SPF composition

Prior to each test of PE1-PE3, each of four PMMA plates treated with the Reference SPF composition were tested to ensure the equipment was maintained within expected limits. The PMMA plate with the reference composition was placed 45.7 cm from the UV beam. The time period to irradiate the PMMA plate with 4 MEDs was determined via the dose controller. The SPF reference PMMA plate was placed in the UV spectrophotometer and the mean MPF values were recorded with 9 measurements at 9 different locations on the PMMA plate for each wavelength over the full UV spectrum (290 to 400 nm). The SPF in vitro value was compared to the in vivo known reference to ensure values are within the expected range. The upper and lower limits were established for the reference composition according to ISO 24444. The measured in vitro SPF value for the Reference SPF composition must be within the range 13.7 to 18.5.

### UV test of benthic pennate diatom compositions.

Each of the five PMMA plates for each of PE1-PE3 were placed 45.7 cm from the UV beam. Five plates of each of PE1-PE3 were placed in the UV spectrophotometer. The mean MPF values were recorded via 9 measurements at 9 different locations on each of the PMMA plates for each wavelength over the full UV spectrum (290 to 400 nm). The MPF values were recorded at 1 nm intervals. The mean values for SPF are shown below in the results section.

### Results

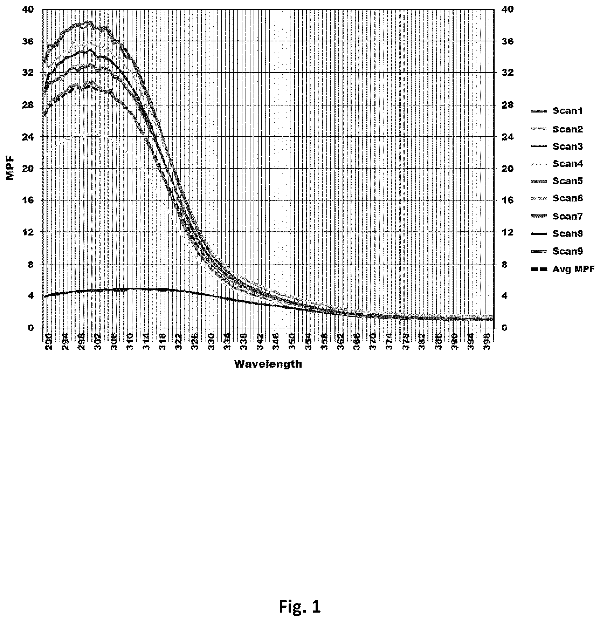

The MPF values obtained for the Reference SPF composition for an example plate are shown in Figure 1.

**Table 3: Mean SPF for Reference SPF composition**

| PMMA Plate no | Mean SPF (9 scans) |
|---|---|
| 1 | 13.18 |
| 2 | 12.88 |
| 3 | 12.25 |
| 4 | 14.56 |
| 5 | 16.99 |
| Mean SPF ± SD | 13.97 ± 1.89 |

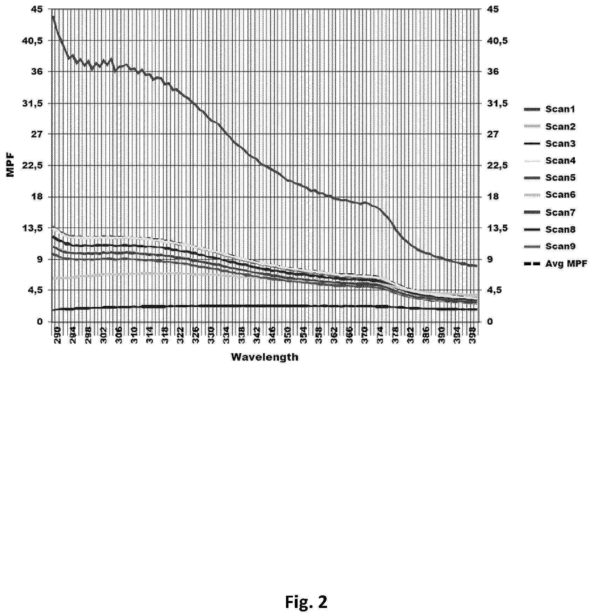

The MPF values obtained for PE1 for an example plate are shown in Figure 2.

**Table 4: Mean SPF for PE1 (0% benthic pennate diatom frustules)**

| PMMA Plate no | Mean SPF (9 scans) |
|---|---|
| 1 | 13.15 |
| 2 | 10.68 |
| 3 | 16.10 |
| 4 | 17.18 |
| 5 | 11.24 |
| Mean SPF ± SD | 13.67 ± 2.89 |

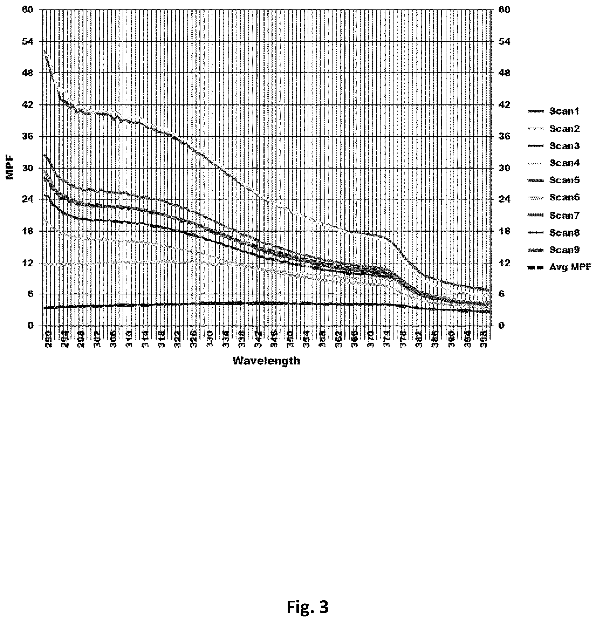

The MPF values obtained for PE2 for an example plate are shown in Figure 3.

**Table 5: Mean SPF for PE2 (0.1% benthic pennate diatom frustules)**

| PMMA Plate no | Mean SPF (9 scans) |
|---|---|
| 1 | 14.54 |
| 2 | 16.99 |
| 3 | 20.30 |
| 4 | 14.40 |
| 5 | 17.59 |
| Mean SPF ± SD | 16.76 ± 2.44 |

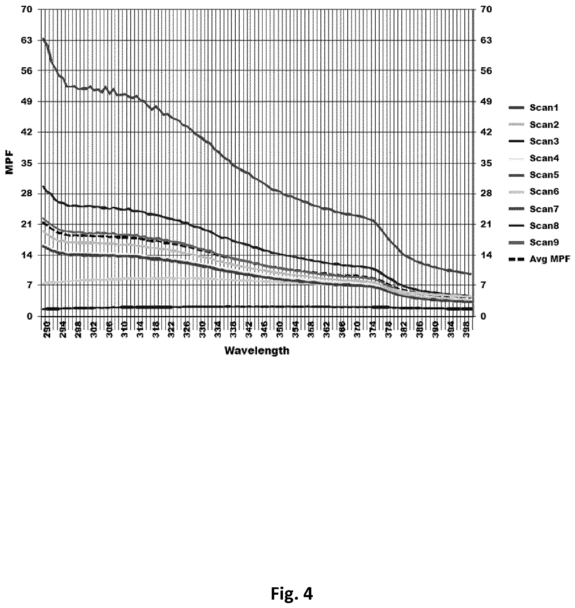

The MPF values obtained for PE3 for an example plate are shown in Figure 4.

**Table 6: Mean SPF for PE3 (0.3% benthic pennate diatom frustules)**

| PMMA Plate no | Mean SPF (9 scans) |
|---|---|
| 1 | 17.39 |
| 2 | 16.52 |
| 3 | 13.06 |
| 4 | 14.69 |
| 5 | 14.90 |
| Mean SPF ± SD | 15.31 ± 1.69 |

**Table 8: Mean SPF for each composition**

| PMMA plate | Mean SPF | Standard Deviation | SPF increase (%) with respect to PE1 |
|---|---|---|---|
| SPF reference | 13.97 | 1.89 | N/A |
| PE1 (0%) | 13.67 | 2.89 | N/A |
| PE2 (0.1%) | 16.76 | 2.44 | 21,99% |
| PE3 (0.3%) | 15.31 | 1.69 | 11,99% |

### Discussion

As can be seen from the above results, compositions comprising benthic pennate diatom frustules have an SPF boosting effect compared to creams without benthic pennate diatom frustules. Compositions comprising benthic pennate diatom frustules in concentrations of 0.1% (PE2) and 0.3% (PE3) have significantly higher mean SPF than the control composition (PE1).

As can be seen from the above table, the standard deviation decreased successively with each increase in the amount of benthic pennate diatoms. This indicates that benthic pennate diatom frustules may have a more consistent boosting effect at increased ratios.

Furthermore, it should be noted that the sunscreen active agent in the specific test was a mineral was present in an amount of 12% in the compositions PE1-PE3. The addition of only 0.1% benthic pennate diatoms provided therefore a remarkable benefit.

Without wishing to be bound by theory, an amount of benthic pennate diatom frustules which can form a monolayer of frustules when applied as a composition may be the optimal amount with respect to SPF boosting effect. This implies that the boosting effect of benthic pennate diatom frustules may not necessarily be greater at significantly increased amounts in the composition. The optimal amount which has the greatest SPF boosting effect, which may be the amount which forms a monolayer when applied to a surface, may depend on the specific surface and the specific composition.

Although, the present invention may have been described above with reference to specific or formulations, it is not intended to be limited to the specific form set forth herein. Rather, the invention is limited only by the accompanying claims.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality.

## Claims

1. A cosmetic composition comprising:
- at least one sunscreen active agent; and,
- benthic pennate diatom frustules.

2. The cosmetic composition according to claim 1, wherein the benthic pennate diatom frustules are an SPF booster.

3. The cosmetic composition according to claim 1 or 2, wherein the composition comprises at least 0.05% (wt.) benthic pennate diatom frustules.

4. The cosmetic composition according to claims 1 to 3, wherein the composition comprises from about 0.05% (wt.) to about 1% (wt.) benthic pennate diatom frustules.

5. The cosmetic composition according to any of claims 1 to 4, wherein the composition comprises from about 0.05% (wt.) to about 0.3% (wt.) benthic pennate diatom frustules.

6. The cosmetic composition according to any of claims 1 to 5, wherein the sunscreen active agent is present in an amount of from 0.1% to 75% (wt.), such as from 1% (wt.) to 50% (wt.).

7. The cosmetic composition according to any of claims 1 to 6, wherein the sunscreen active agent is at least one organic sunscreen active agent, or at least one inorganic sunscreen active agent, the inorganic sunscreen agent selected from the list comprising: titanium dioxide, zinc oxide, iron oxide, zirconium oxide, cerium oxide; or, a combination thereof.

8. The cosmetic composition according to any of claims 1 to 7, wherein the cosmetic composition is provided in an acceptable cosmetic carrier medium, and wherein the acceptable cosmetic carrier medium is water or oil.

9. The cosmetic composition according to any of claims 1 to 8, wherein the cosmetic composition is provided in powdered form.

10. The composition according to any of claims 1 to 9, wherein the frustules are extracted from cultured and harvested benthic pennate diatoms.

11. A method of boosting the SPF of a sunscreen composition comprising:
- including benthic pennate diatom frustules.

12. The method according to claim 11, wherein the benthic pennate diatom frustules are included in an amount from about 0.05% to about 1% by weight of the composition.

13. The method according to claim 11 or 12, wherein the sunscreen has an SPF that is at least 10% higher than a corresponding sunscreen in which benthic pennate diatoms frustules are not present.

14. A method of producing a sunscreen composition comprising:
- providing a sunscreen active agent to a cosmetic carrier composition, and
- providing benthic pennate diatom frustules.

15. The method according to claim 14, wherein the benthic pennate diatom frustules are provided at an amount of from about 0.05% to about 1% by weight of the composition.

## Patentansprüche

1. Kosmetische Zusammensetzung, umfassend:
- mindestens einen Sonnenschutzwirkstoff; und
- benthische pennate Kieselalgenfrusteln.

2. Kosmetische Zusammensetzung nach Anspruch 1, wobei die benthischen pennaten Kieselalgenfrusteln ein SPF-Booster sind.

3. Kosmetische Zusammensetzung nach Anspruch 1 oder 2, wobei die Zusammensetzung mindestens zu 0,05 % (Gew.-%) benthische pennate Kieselalgenfrusteln umfasst.

4. Kosmetische Zusammensetzung nach den Ansprüchen 1 bis 3, wobei die Zusammensetzung von etwa 0,05 % (Gew.) bis etwa 1 % (Gew.) benthische pennate Kieselalgenfrusteln umfasst.

5. Kosmetische Zusammensetzung nach einem der Ansprüche 1 bis 4, wobei die Zusammensetzung von etwa 0,05 % (Gew.) bis etwa 0,3 % (Gew.) benthische pennate Kieselalgenfrusteln umfasst.

6. Kosmetische Zusammensetzung nach einem der Ansprüche 1 bis 5, wobei der Sonnenschutzwirkstoff in einer Menge von 0,1 % bis 75 % (Gew.), wie von 1 % (Gew.) bis 50 % (Gew.), vorhanden ist.

7. Kosmetische Zusammensetzung nach einem der Ansprüche 1 bis 6, wobei der Sonnenschutzwirkstoff mindestens ein organischer Sonnenschutzwirkstoff oder mindestens ein anorganischer Sonnenschutzwirkstoff ist, wobei der anorganische Sonnenschutzwirkstoff aus der Liste ausgewählt ist, umfassend: Titandioxid, Zinkoxid, Eisenoxid, Zirkoniumoxid, Ceroxid; oder eine Kombination davon.

8. Kosmetische Zusammensetzung nach einem der Ansprüche 1 bis 7, wobei die kosmetische Zusammensetzung in einem akzeptablen kosmetischen Trägermedium bereitgestellt wird und wobei das akzeptable kosmetische Trägermedium Wasser oder Öl ist.

9. Kosmetische Zusammensetzung nach einem der Ansprüche 1 bis 8, wobei die kosmetische Zusammensetzung in Pulverform bereitgestellt ist.

10. Zusammensetzung nach einem der Ansprüche 1 bis 9, wobei die Frusteln aus kultivierten und geernteten benthischen pennaten Kieselalgen extrahiert werden.

11. Verfahren zum Erhöhen des Lichtschutzfaktors einer Sonnenschutzzusammensetzung, umfassend:
- einschließlich benthischer pennater Kieselalgenfrusteln.

12. Verfahren nach Anspruch 11, wobei die benthischen pennaten Kieselalgenfrusteln in einer Menge von etwa 0,05 Gew.-% bis etwa 1 Gew.-% der Zusammensetzung eingeschlossen sind.

13. Verfahren nach Anspruch 11 oder 12, wobei das Sonnenschutzmittel einen Lichtschutzfaktor aufweist, der mindestens 10 % höher ist als der eines entsprechenden Sonnenschutzmittels, in dem keine benthischen pennaten Kieselalgenfrusteln vorhanden sind.

14. Verfahren zum Herstellen einer Sonnenschutzzusammensetzung, umfassend:
- Bereitstellen eines Sonnenschutzwirkstoffs für eine kosmetische Trägerzusammensetzung und
- Bereitstellen von benthischen pennaten Kieselalgenfrusteln.

15. Verfahren nach Anspruch 14, wobei die benthischen pennaten Kieselalgenfrusteln in einer Menge von etwa 0,05 Gew.-% bis etwa 1 Gew.-% der Zusammensetzung bereitgestellt werden.

## Revendications

1. Composition cosmétique comprenant :
- au moins un agent actif de protection solaire ; et,
- des frustules de diatomées benthiques pennées.

2. Composition cosmétique selon la revendication 1, dans laquelle les frustules de diatomées benthiques pennées sont un amplificateur de SPF.

3. Composition cosmétique selon la revendication 1 ou 2, dans laquelle la composition comprend au moins 0,05 % (en poids) de frustules de diatomées benthiques pennées.

4. Composition cosmétique selon les revendications 1 à 3, dans laquelle la composition comprend d'environ 0,05 % (en poids) à environ 1 % (en poids) de frustules de diatomées benthiques pennées.

5. Composition cosmétique selon l'une quelconques des revendications 1 à 4, dans laquelle la composition comprend d'environ 0,05 % (en poids) à environ 0,3 % (en poids) de frustules de diatomées benthiques pennées.

6. Composition cosmétique selon l'une quelconque des revendications 1 à 5, dans laquelle l'agent actif de protection solaire est présent en une quantité allant de 0,1 % à 75 % (en poids), par exemple de 1 % (en poids) à 50 % (en poids).

7. Composition cosmétique selon l'une quelconque des revendications 1 à 6, dans laquelle l'agent actif de protection solaire est au moins un agent actif de protection solaire organique ou au moins un agent actif de protection solaire inorganique, l'agent de protection solaire inorganique étant choisi dans la liste comprenant : le dioxyde de titane, l'oxyde de zinc, l'oxyde de fer, l'oxyde de zirconium, l'oxyde de cérium ; ou une combinaison de ceux-ci.

8. Composition cosmétique selon l'une quelconque des revendications 1 à 7, dans laquelle la composition cosmétique est fournie dans un support cosmétique acceptable, et dans laquelle le support cosmétique acceptable est de l'eau ou de l'huile.

9. Composition cosmétique selon l'une quelconque des revendications 1 à 8, dans laquelle la composition cosmétique est fournie sous forme de poudre.

10. Composition selon l'une quelconque des revendications 1 à 9, dans laquelle les frustules sont extraits de diatomées benthiques pennées cultivées et récoltées.

11. Procédé d'augmentation du SPF d'une composition de protection solaire comprenant :
- y compris les frustules de diatomées benthiques pennées.

12. Procédé selon la revendication 11, dans lequel les frustules de diatomées benthiques pennées sont incluses dans une quantité d'environ 0,05 % à environ 1 % en poids de la composition.

13. Procédé selon la revendication 11 ou 12, dans lequel l'écran solaire a un SPF supérieur d'au moins 10 % à celui d'un écran solaire correspondant dans lequel les frustules de diatomées pennées benthiques ne sont pas présentes.

14. Procédé de production d'une composition de protection solaire comprenant :
- l'ajout d'un agent actif de protection solaire à une composition de support cosmétique, et
- la fourniture des frustules de diatomées benthiques pennées.

15. Procédé selon la revendication 14, dans lequel les frustules de diatomées benthiques pennées sont fournies en une quantité allant d'environ 0,05 % à environ 1 % en poids de la composition.
